# EUROPEAN PATENT SPECIFICATION

(11) **EP 4 444 297 B1**
(45) Date of publication and mention of the grant of the patent: **09.12.2026**
(21) Application number: 22826197.0
(22) Date of filing: 17.11.2022
(51) Int. Cl.: A61K 31/352, A61K 31/7048, A61K 31/727, A61K 45/06, A61P 9/00

(54) **ORAL FORMULATION BASED ON A PRE-ESTABLISHED ASSOCIATION OF SULODEXIDE AND FLAVONOIDS FOR THE TREATMENT OF CHRONIC VENOUS INSUFFICIENCY AND PREVENTION OF COMPLICATIONS THEREOF**
ORALE FORMULIERUNG AUF BASIS EINER VORGEGEBENEN KOMBINATION AUS SULODEXID UND FLAVONOIDEN ZUR BEHANDLUNG CHRONISCHER VENENINSUFFIZIENZ UND PRÄVENTION VON KOMPLIKATIONEN DAVON
FORMULATION ORALE BASÉE SUR UNE ASSOCIATION PRÉ-ÉTABLIE DE SULODEXIDE ET DE FLAVONOÏDES POUR LE TRAITEMENT D'UNE INSUFFISANCE VEINEUSE CHRONIQUE ET LA PRÉVENTION DE COMPLICATIONS DE CELLE-CI

(30) Priority: 07.12.2021 IT 202100030815
(43) Date of publication of application: 16.10.2024
(73) Proprietor: Omikron Italia S.r.L., 00189 Rome (IT)
(72) Inventor: VIRNO, Cristiano, 00197 Roma (IT)
(74) Representative: Di Giovine, Paolo
(86) International application number: PCT/IB2022/061071
(87) International publication number: WO 2023/105325

(56) References cited:
- GONZÁLEZ OCHOA ALEJANDRO: "Sulodexide and phlebotonics in the treatment of venous ulcer", INTERNATIONAL ANGIOLOGY, vol. 36, no. 1, 1 January 2017 (2017-01-01), IT, XP055940496, ISSN: 0392-9590, DOI: 10.23736/S0392-9590.16.03718-4
- CORSALE ITALO ET AL: "Flavonoid mixture (diosmin, troxerutin, rutin, hesperidin, quercetin) in the treatment of I-III degree hemorroidal disease: a double-blind multicenter prospective comparative study", INTERNATIONAL JOURNAL OF COLORECTAL DISEASE, SPRINGER VERLAG, BERLIN, DE, vol. 33, no. 11, 22 June 2018 (2018-06-22), pages 1595 - 1600, XP036613707, ISSN: 0179-1958, [retrieved on 20180622], DOI: 10.1007/S00384-018-3102-Y
- LICHOTA ANNA ET AL: "Therapeutic potential of natural compounds in inflammation and chronic venous insufficiency", EUROPEAN JOURNAL OF MEDICINAL CHEMISTRY, vol. 176, 15 August 2019 (2019-08-15), pages 68 - 91, XP085707318, ISSN: 0223-5234, DOI: 10.1016/J.EJMECH.2019.04.075
- ANDREOZZI ET AL: "Sulodexide in the treatment of chronic venous disease", AMERICAN JOURNAL OF CARDIOVASCULAR DRUGS, ADIS INTERNATIONAL, NZ, vol. 12, no. 2, 1 April 2012 (2012-04-01), pages 73 - 81, XP008175471, ISSN: 1175-3277, [retrieved on 20120817], DOI: 10.2165/11599360-000000000-00000

## Description

The present invention relates to an oral composition comprising sulodexide and one or more flavonoids as active principles, and to said oral composition for use in the treatment of a venous disease or disorders or symptoms associated with or caused by said venous disease in particular, in the treatment of the chronic venous insufficiency (CVI) and/or prevention of complications thereof.

### STATE OF PRIOR ART

The vascular diseases are among the most widespread diseases in the world. Thereamong, the chronic venous insufficiency (CVI) of the lower limbs represents one of the diseases with higher incidence and prevalence in the female gender.

CVI is an "outflow disease", consequent to structural anomalies involving the walls of veins, of dilative type (varices) or obstructive type, the latter including, among the various symptoms, deep vein thromboses (DVT), superficial vein thromboses (SVT), and post-thrombotic syndromes. CVI clinical pictures are very variegated and the intensity and gravity of the disease can be extremely variable, by determining the onset of alterations at aesthetical level only (such as reticular varices, telangiectasia), until considerable conditions of tissue damage (such as lipodermatosclerosis, trophic ulcers).

Several factors, for example predisposing hereditary-constitutional factors or triggering factors such as sedentary lifestyle, overweight, hormones and pregnancies, concur in determining the onset of CVI. Such factors can trigger a complex physiopathological mechanism which, through a damage to the venous and/or lymphatic drainage system, causes an insufficiency in the venous system, by compromising the operation thereof as centripetal draining organ. This phenomenon may happen due to anomalies of the segmentary valve structures (for example post-thrombotic syndrome with destruction of the valve system), and after dilatation of the vein walls (for example varices) which do not allow anymore an adequate valve competence.

From the segmentary valve insufficiency primitive various syndromes as well as secondary varices with insufficiency of the deep vein circulation (post-thrombotic syndrome) may originate. From the resulting vein hypertension, underlying all phlebo-lymphostatic pictures involving above all the districts more involved by the force of gravity (for example perimalleolar area), the interstitial imbibition as well as more common pathological alterations may originate: from oedema to the thrombotic and phlebitis processes, to lipodermatosclerosis and to the trophic lesions.

CVI is characterized by variations in the parietal tension and vein pressure which are responsible for an alteration in the micro-vasculo-tissue homeostasis, with consequent release of the inflammation mediators and increase in the vasal permeability. In the last years lots of progresses have been obtained in the comprehension of CVI physiodisease and alterations at cellular or ultrastructural level which trigger the macroscopic anomalies which are clinically found. In the centre of the pathological process an inflammatory phenomenon is found which, through alterations in the usual endothelial coating of the venous walls (glycocalyx), due to stasis, *shear stress,* venous hypertension and hematic hyperviscosity, involves first of all the adhesion and then then passage of the white blood cells by diapedesis in the sub-endothelial layer. The so-activated white blood cells release a series of "inflammatory" factors which start a chemotactic process amplifying the phlogosis itself and the additional release of substances mediating local "harmful" substances. Such inflammatory processes coexist with parallel hemodynamic phenomena (reflux, venous hypertension, interstitial imbibition) and at last they synergically determine the common clinical consequences in terms of symptoms (heaviness and heat, swelling, pain) as well as signs (oedema, blush, varices, dermo-hypodermitis, phlebitis).

Once a correct anamnestic collection and specific diagnostics, including instrumental analysis (for example, hemodynamic cartography with eco-colour doppler) have been performed, the currently available main strategies for therapeutically treating CVI provide to adopt adequate standards of conduct aimed at guaranteeing a better lifestyle to the patient. The use of a graduated and decreasing elastic compression, especially to face the moments of obliged sedentary lifestyle in the everyday life, together with a supporting medical therapy with the purpose of obtaining a condition of clinical and hemodynamic stabilization of the disease, surely represent for the patient the main strategy for treating CVI. The synergic cooperation between the adoption of adequate standards of conduct, the elastic compression and the medical therapy allows to obtain a stability condition of the disease, by avoiding the progression thereof and the development of complications. The same treatment strategy, in a more aggressive form, is generally proposed even in a subsequent stage of the disease, with the purpose of decreasing the intensity thereof, limiting the progression and onset of complications, as well as remedying to the produced aesthetical discomfort.

The CVI medical therapy has developed over the last decades thanks to several experimental and clinical studies which have allowed to elucidate the mechanisms underlying the disease, for example venous hypertension, stasis, inflammatory phenomena, tissue remodelling, degradation of the matrix proteins.

González Ochoa A. Sulodexide and phlebotonics in the treatment of venous ulcer. Int Angiol. 2017 Feb;36(1):82-87. doi: 10.23736/S0392-9590.16.03718-4. Epub 2016 Jun 16. PMID: 27310527 investigates whether the combined administration of both suledoxide and diosmin-hesperidin micronized purified flavonoids (DH) is better than DH alone in the treatment of venous ulcers.

Notwithstanding the several progresses obtained in the comprehension of the mechanisms underlying the onset of the vascular diseases, such as CVI, the need is still much felt for detecting effective strategies of therapeutic treatment allowing to guaranteeing not only a better quality of life and a stabilization of the clinical picture of the patient, but even a better capability of preventing or speeding up the healing of the complications associated therewith.

### SUMMARY OF THE INVENTION

The object underlying the present invention is to provide an oral composition particularly effective in the treatment of vascular diseases and in the prevention of the complications thereof. The present invention to this purpose provides an oral composition comprising sulodexide and one or more flavonoids. According to a preferred embodiment, the composition of the invention comprises one or more flavonoids selected among diosmin, hesperidin, troxerutin, oxerutin, quercitin and hesperidin.

The authors of the present invention found that the specific combination of the active principles sulodexide and one or more flavonoids, in oral formulation, results to be surprisingly more effective in the treatment of vascular diseases, such as CVI, and in the prevention of complications associated therewith, than the single active principles.

The action of the composition, the present invention relates to, was evaluated by *in vitro* and *in vivo* tests which, in fact, highlighted that this results to be particularly effective in the treatment of vascular diseases thanks to the synergic action of its components used in association rather than the single active ingredients.

In particular, the results obtained by the inventors on an animal model of CVI, shown in the experimental section of the present description (Example 2), demonstrated the surprising capability of the composition, the invention relates to, to restore the microvascular alterations in the treated animal, with a higher effectiveness than the one determined for the single active principles present in the composition, administered singularly.

Moreover, the data obtained from the *in vitro* and *in vivo* tests performed by the authors of the invention and shown in the experimental section of the present description surprisingly demonstrated that, thanks to the synergic action between the active principles present therein, the compositions, the present invention relates to, allow to obtain a greater effectiveness in the treatment of vascular diseases than the active principles administered singularly, even if the single active principles are present in the composition with lower dosages than the dosages used in the administration of the single molecules.

The association between sulodexide and one or more flavonoids in oral formulation in the medical treatment of CVI, then, represents an effective therapeutic solution both in the early stages and in the more serious stages of the disease. Advantageously, the use of the compositions according to the present invention is advisable not only in a purely conservative step of the disease, in association with adequate living standards and graduated elastic compression, but even in a step of "aggressive" management of the disease, as adjuvant; in other terms, the compositions, the invention relates to, have a huge potential from a "conservative" and "ablative" point of view in the treatment of the varicose syndromes and in managing trophic lesions, for the treatment thereof the use of a pharmacological therapy allows to speed up the ulcer resolution time.

An effective medical therapy such as the one proposed by the authors of the present invention can fully support adequate standards of conduct and the application of graduated elastic compression already at the beginning of the chronic venous insufficiency (C0s-C3). As ally of the "direct" ablative or conservative therapies of the varicose syndromes and even in the more advanced stages which characterize the chronic venous insufficiency (C4-C6), such medical therapy can favour a better quality of life, a stabilization of the clinical picture, a better prognosis for the patient and a significant help in speeding up the healing of the venous ulcers.

The combination of sulodexide and one or more flavonoids, in particular diosmin, in the same oral administration, then allows to widen the spectrum of action and effectiveness of the medical therapy for the vascular diseases and promises to become the *"best medical therapy",* even with a better therapeutic adherence for the patient.

Therefore, the present invention relates to:
- an oral composition comprising sulodexide and one or more flavonoids;
- an oral composition according to any one of the herein described embodiments, for use in the treatment of vascular diseases and/or in the prevention of complications associated therewith.

Additional advantages, as well as the features and use modes of the present invention, will result evident from the following detailed description of some preferred embodiments.

### DETAILED DESCRIPTION OF FIGURES

Figure 1. Effect of the various treatments specified in Example 1 on hyperpermeability induced with the vascular endothelial growth factor (VEGF) of the Monolayer consisting of human microvascular endothelial cells (HMVEC).
Figure 2. Effect of the various treatments specified in Example 1 on hyperpermeability induced with VEGF of the Monolayer consisting of human umbilical vein endothelial cells (HUVEC).
Figure 3. Effect of the various treatments specified in Example 2 on venous inflammation. Evaluation of the leukocytic *rolling:* the leukocytes were marked with rodamin 6-G administered before observation intravenously.
Figure 4. Effect of the various treatments specified in Example 2 on the functional capillary density (meant as the blood speed in the micro-vessels, correlated to the distance travelled by a red blood cell).
Figure 5. Effect of the various treatments specified in Example 2 on the venular diameter evaluated through intravital microscopy.
Figure 6. Effect of the various treatments specified in Example 3 on the percentage of restoring maximum venous contractility to the noradrenergic stimulus under conditions of hyperthermia (41°C).

### GLOSSARY

The terms used in the present description are as generally understood by the person skilled in the art, unless otherwise indicated.

The term "chronic venous insufficiency", in short as CVI, in the context of the present invention, has the meaning commonly used in the medical practice. The chronic venous insufficiency is a disease characterized by a progressive deterioration in the vascular functionality, especially of the lower limbs, which in the long term determines the onset of pain, oedema, inflammation. Such disease is characterized by variations in the parietal tension and in the venous pressure responsible for an alteration in the micro-vascular-tissue homeostasis, with consequent release of the inflammation mediators, increase in the vasal permeability and decrease in the venous tone.

For a standardized description of the clinical picture and of the CVI symptoms, one commonly refers to a clinical classification system indicated by the acronym "CEAP" (Clinical, Etiology, Anatomy, Pathophysiology).

According to such system, the different stages of the disease can be indicated as follows: C0s: symptoms without evidence of disease; C1: reticular veins and teleangiectasia; C2: varices; C3: oedema C4: lipodermatosclerosis; C5: healed ulcer; C6: active ulcer.

The expression "composition for oral use", in the context of the present invention, relates to a composition for oral administration to a subject requiring it, and then it comprises a composition according to any one of the herein described embodiments in any form known to a person skilled in the art which results to be suitable for an oral administration.

### DETAILED DESCRIPTION OF THE INVENTION

As mentioned above, the present invention relates to an oral composition comprising sulodexide and one or more flavonoids.

As clearly highlighted by the results of the studies shown in the experimental section of the present description (Example 2 and 3), the inventors found and demonstrated that the composition, the present application relates to, has a significant effectiveness in the treatment of the microvascular alterations, deriving from the action selectivity of its active principles, as well as from the synergy of their combination.

Sulodexide is a biological drug of natural origin purified by porcine intestinal mucosa, consisting of a defined mixture of glycosaminoglycans (GAGs) consisting by 80% of heparin with low molecular weight and by the remaining 20% of dermatan sulfate (DS). It is characterized by a complex pharmacological profile with several effects on the vascular system: antithrombotic, profibrinolytic, direct anti-inflammatory on the metalloproteinases 2 and 9 (MMP-2 and MM9-9), structural for reintegration of glycocalyx and protective for endothelium. The sulodexide usable in a composition according to the present invention can be produced according to any one of the techniques known to a person skilled in the art, and it can be present in the composition even in form of a pharmaceutically acceptable salt, solvate or hydrate thereof.

The flavonoids represent a class of chemical compounds of natural origin. These show a demonstrated direct action on the interstitial connective and have marked effects on the parietal tone, on the vasal permeability and on the inflammatory process.

The most used flavonoids substantially belong to the group of flavones and derivatives. Therefore, according to a preferred aspect, one or more flavonoids usable in a composition according to any one of the herein described embodiments are flavones or derivatives thereof, for example by direct belonging (for example diosmin, flavone) or indirect belonging as indeed derivatives (for example hesperidin, reduced flavone; troxerutin, hydroxy-flavone).

The flavonoids usable in the compositions, the present invention relates to, even if belonging, each one, to a specific subfamily can be traced back to the family of the "flavones and/or derivatives".

The chemical structure of the flavones comprises a skeleton C₁₅ of carbon atoms consisting of two aromatic rings and one heterocyclic ring; one aromatic ring (ring A) results to be condensed with the heterocyclic ring (ring C) and the third one (aromatic ring B) connected to the ring C in position 2.

The flavones are soluble in water and ethanol and show a characteristic yellow colour if dissolved in alkaline solution.

The expressions "derivatives" or "flavonic derivative" as used in the context of the present invention, then relate to compounds belonging to the family of the flavonoids, characterized by or obtained starting from chemical modifications and/or replacements of the base chemical structure of the flavones.

One or more flavonoids usable in a composition according to any one of the herein described embodiments can be obtained starting from vegetable material, for example plants, by using any one of the techniques known to a person skilled in the art.

According to an aspect of the present invention, said one or more flavonoids are preferably selected among diosmin, hesperidin, troxerutin, oxerutin, quercitin and hesperidin, preferably among diosmin, hesperidin and troxerutin.

Diosmin is a poly-hydroxylated flavone having eight hydroxyl groups, two thereof phenolic, with ubiquitous distribution in the vegetable world, which has the following structure:

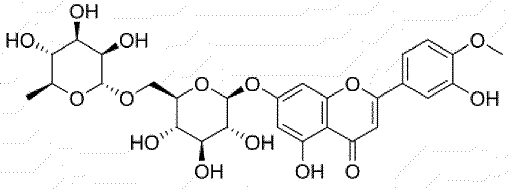

The fruits of genus *Citrus* (lemons, oranges, grapefruits etc.), Bucco leaves Bucco (*Barosma betulina*) and the flowering tops of Ruta (*Ruta graveolens*) contain significant concentrations of diosmin.

Hesperidin is a flavonic derivative belonging to the subfamily of the flavanones, or a type of flavonoid which can be found especially inside the fruits of the citrus fruits, characterized by strong antioxidant, capillarotropic and vasoprotective activity, having the following structure:

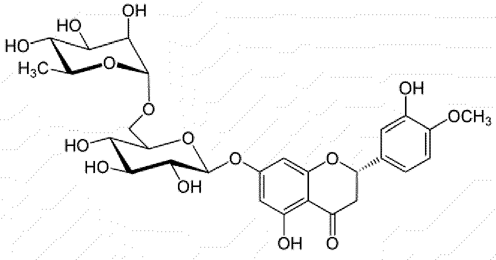

Chemically, troxerutin is an hydroxyethylrutoside, derivative from rutin, having the following structure:

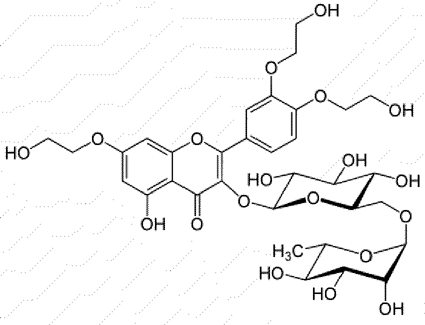

Such compound is a flavonic derivative which can be isolated from *Sophora japonica,* the Japanese pagoda tree, but it is present even in tea, in coffee, in cereals and in many varieties of fruit and vegetable.

Diosmin, hesperidin and/or troxerutin usable in any one of the herein described compositions, can be of synthetic origin or of natural origin. In the compositions of the present invention plant extracts or plant parts containing one or more flavonoids such as for example diosmin, hesperidin and/or troxerutin, for example extracts of plant comprising a defined title of flavonoids, preferably expressed as percentages by weight with respect to the total extract, could also be used. In the prior art plant extracts containing one or more flavonoids such as for example diosmin, hesperidin troxerutin, oxerutin, quercitin and/or hesperidin are known.

The person skilled in the art could adapt the amount of sulodexide and of one or more flavonoids used in the preparation of the oral compositions of the invention depending upon needs.

According to a preferred aspect of the invention, sulodexide is present in a composition according to any one of the herein described embodiments in an amount comprised between 21 mg and 29 mg preferably equal to 25 mg.

The concentration of sulodexide can be expressed in units releasing lipoprotein lipase (or lipasemic units): such unit measuring the biological activity of sulodexide is based upon the capability of this active principle, injected intravenously in rats, to release in blood the clearing factor or β lipoprotein lipase, a lytic enzyme only attacking the triglycerides linked to proteins. 1 mg of sulodexide corresponds to 10 ULS. Preferably, sulodexide is present in a composition according to any one of the herein described embodiments, in an amount equal to 250 ULS (lipasemic units).

According to an aspect of the invention, said one or more flavonoids, and in particular said one or more flavones and/or derivatives thereof, are present in a composition according to any one of the herein described embodiments in an amount comprised between 380 mg and 520mg preferably equal to 450 mg.

The inventors detected the optimum pro-dose (or per single dosage unit) dosages of each substance of the composition of the invention to obtain synergic therapeutic effects. In particular, the best effects are obtained when sulodexide is present in an amount equal to 25 mg and said one or more flavonoids are present in an amount equal to 450 mg.

From the performed experimentation, the inventors found that the combination of sulodexide and diosmin in formulation for oral use results to be surprisingly more effective than the single active principles administered singularly.

A preferred embodiment according to the invention then relates in particular to an oral composition comprising sulodexide and diosmin. Preferably this preferred composition comprises sulodexide in an amount equal to 25 mg by weight and diosmin in an amount equal to 450 mg by weight.

According to a preferred aspect, the compositions of the invention comprise a pre-established association of sulodexide and one or more flavonoids according to any one of the herein described embodiments. By pure way of example, the compositions of the invention can be obtained through at least a step of mixing sulodexide and one or more flavonoids according to any one of the herein described embodiments so as to obtain a pre-established association of the above-mentioned active principles.

The present invention also relates to an oral composition according to any one of the herein described embodiments, further comprising one or more suitable excipients.

Suitable excipients can be selected among those usually known in the state of art and include, without being limited thereto: carriers (for example soya bean oil), diluents, lubricants (for example magnesium stearate, stearic acid, waxes), dispersants, surface active agents or surfactants (for example sodium lauryl sulfate and polysorbates), flavouring agents, adsorbents (for example silica gel, talcum, starch, bentonite, kaolin), glidants and anti-adherent agents (for example talcum, colloidal silica, maize starch, silicon dioxide), colouring agents (for example iron oxides), opacifiers (for example titanium oxide), antioxidants, binders (for example gums, starch, gelatine, derivatives of cellulose, sucrose, alginate sodium), disaggregating agents (starch, microcrystalline cellulose, alginic acid, crospovidone), plasticizing agents or plasticizers (for example ethylcellulose and other cellulose derivatives, acrylates and methacrylates, glycerol and sorbitol), preservatives (for example parabens, sulfur dioxide, ethyl p-hydroxybenzoate, propyl-p-hydroxybenzoate), viscosifiers, thickeners (for example beeswax), emulsifiers, humectants, wetting agents, chelating agents and mixtures thereof.

According to a preferred aspect of the invention, said sulodexide and said one or more flavonoids are the active principles of the composition in any one of the herein described embodiments.

Under the term "active principles", in the context of the present invention, one refers to the fact that sulodexide and said one or more flavonoids are present inside a composition according to the invention in an effective amount to obtain the wished therapeutic effect in a subject who is subjected to a therapeutic treatment providing the administration of the above-mentioned composition.

A preferred embodiment according to the invention in particular relates to an oral composition according to any one of the herein described embodiments, wherein said sulodexide and said one or more flavonoids are the only active principles of the composition.

According to an additional aspect of the invention, the compositions of the invention however could optionally include even one or more additional active principles.

The compositions of the invention can be implemented in any pharmaceutically acceptable form known to a person skilled in the art, provided that it is suitable for an administration of oral type.

The compositions of the invention can be for example in solid, liquid or gel form, for example, in the form of tablet, hard capsule, soft capsule, suspension, emulsion, solution.

A preferred embodiment according to the present invention relates to a composition in form of soft capsule, having the quali-quantitative composition as shown in the following table 1:

**Table 1 - Example of composition according to the invention**

| **Active principles** | |
|---|---|
| Sulodexide (glucuronyl glucosamine glycane sulfate) | 25 mg |
| Diosmin | 450 mg |

| **Excipients** | |
|---|---|
| *Filling mixture* | |
| Surfactant | for example, sodium lauryl sulfate |
| Carrier | for example, Soy bean Oil, refined |
| Thickening agent | for example, Beeswax, yellow |

| *Casing* | |
|---|---|
| Plasticizing agent | for example, glycerol |
| Casing material | for example, Gelatine |
| Preservative agent | for example, ethyl p-hydroxybenzoate |
| Preservative agent | for example, propyl-p-hydroxybenzoate |
| Opacifier | for example, titanium dioxide |
| Colouring agent (one or more) | for example, iron oxide |

According to an aspect of the present invention, the compositions in any one of the herein described embodiments are pharmaceutical compositions.

As previously mentioned, the present invention also relates to an oral composition according to any one of the herein described embodiments, for use in the treatment of vascular diseases and/or in the prevention of complications associated with or caused by these diseases, in particular in the treatment of chronic venous insufficiency and/or in the prevention of its complications (comprising dyschromia until venous ulcers) and/or symptoms or disorders associated with or caused by this disease (thereamong the post-thrombotic syndrome).

According to an aspect of the present invention, the compositions according to any one of the herein described embodiments are oral compositions for use in the treatment of chronic venous insufficiency of legs and/or of symptoms or disorders associated with or caused by this disease and/or in the prevention of the complications associated with or caused by this disease. The terms "symptoms" or "disorders" associated with or caused by chronic venous insufficiency, in the context of the present invention, also comprise complications associated with or deriving from this disease, thereamong symptoms and/or disorders as exemplified hereinafter.

Examples of symptoms, disorders, or complications which can be associated with vascular diseases, and in particular with the chronic venous insufficiency, or caused by these diseases, are selected from: oedema, motor difficulty, burning sensation, itch, paresthesia or tingling, pain, muscle cramps, feeling of swelling, feeling of heaviness at lower limbs, restless leg syndrome, leg fatigue, postphlebitic syndrome, stasis dermatitis and blotches, thrombotic and phlebitic processes, lipodermatosclerosis, trophic lesions. The capability of the compositions according to any one of the herein described embodiments of treating symptoms or disorders caused by chronic venous insufficiency as exemplified in the present description and/or of preventing the onset of their complications, is the result of the synergic action of the active principles in association in the above-mentioned compositions. In fact, the compositions, the present invention relates to, by determining an effective reduction in vasal hyperpermeability and infiltration by inflammatory molecules, as well a restoration of the venous tone - as it will be clearly highlighted hereinafter -, remove or contribute in removing, actually, a cause or contributing cause relevant for the onset of the complications or symptoms at issue, then by allowing to prevent and/or treat such disorders or at least helping necessarily the treatment of such disorders and/or the prevention of their complications.

The treatment or prevention of the complications according to the present invention comprise at least a step of administering the composition according to any one of the herein described embodiments to a subject requiring it. Subjects suitable to be subjected to a treatment with a composition according to the present invention, in particular are subjects therefore a vascular disease was diagnosed, or subjects therefor a high risk of developing a vascular disease was determined, for example any one of the diseases shown in the text of the present description and/or in the claims, as well as symptoms or disorders associated therewith or caused thereby.

The present invention also relates to an oral composition according to any one of the herein described embodiments for use in a method for the treatment of vascular diseases and the prevention of the complications associated with or caused by these diseases, and in particular of CVI or of symptoms, complications or disorders associated with or caused by this disease, which method comprises at least a step of administering by oral route said composition to a subject requiring it.

The dosage and the therapeutic regime of the oral composition according to the present invention could be established by the treating physician based upon weight, health state, gender and age of the subject to be treated, as well as modified over time based upon the subject's response to the therapeutic treatment.

Therefore, the dosage of the single active principles in an oral composition according to the invention can be adapted, even during the period of taking the composition of the invention, depending upon the results obtained over time. According to an aspect of the invention, an oral composition according to any one of the herein described embodiments could be administered to a subject requiring it in one or more doses, with a daily dosage comprised between 475 and 950 mg/day.

Preferably, the oral compositions of the invention will be administered to a subject requiring it, for example, at least once a day, preferably twice a day.

The oral compositions the present invention relates to, could be prepared by mixing the selected active principles with other possible excipients as it is known to the person skilled in the art.

A therapeutic method for the treatment of vascular diseases and/or the prevention of the complications associated therewith, in particular of chronic venous inflammation and symptoms or disorders associated therewith or caused thereby, is also herein described, which method comprises the administration of an oral composition according to any one of the herein described embodiments in a therapeutically effective dosage to a subject requiring it.

The administration of an oral composition according to the invention can be repeated several times a day and the composition can be provided already under the form of single doses or with a dispenser of single doses.

A "therapeutically effective dosage" means a dosage which is effective in therapy, or an amount sufficient to provide a wished therapeutic effect. A dosage which is effective in therapy is an amount which produces one or more wished biological activities during the treatment or the prevention of a disease. In the present case during the treatment of a vascular disease and the prevention of complications thereof, in particular chronic venous inflammation or a symptom or disorder associated therewith or caused thereby. Examples of disorders or symptoms associated with or caused by chronic venous insufficiency are those already defined above in relation to the medical use of the composition of the invention.

Examples are shown below having the purpose of better illustrating the methods detected in the present description, such examples are in no way to be considered as a limitation of the preceding description and of the subsequent claims.

### EXAMPLES

### Example 1. In vitro studies on human micro and macro-vascular cellular cultures

The direct effect on the vasal permeability of three compositions comprising Sulodexide + flavonoids was evaluated *in vitro:*
(i) Sulodexide + Diosmin (Sul + Dios),
(ii) Sulodexide + Hesperidin (Sul+ Esp);
(iii) Sulodexide + Troxerutin (Sul + Trox).

All tested compositions (i)-(iii) comprise a concentration of sulodexide equal to 0.25% and a concentration of diosmin, hesperidin or troxerutin, respectively, equal to 4.5% by weight with respect to the total weight of the composition.

The action of the compositions (i)-(iii) was compared with the action of the active principles Diosmin, Hesperidin, Troxerutin, 8% by weight, and glucosaminoglycan Sulodexide, 0.5% by weight, administered singularly.

The direct effect on the vasal permeability was evaluated by using endothelial cellular cultures miming the continuous mono-layer of the endothelium of the human vessels. Two cellular models were used: the human micro-vascular endothelial cells (HMVEC) to simulate the vessels of the microcirculation, and the human umbilical vein endothelial cells (HUVEC) to study the vasal endothelium. The cells of the lines HMVEC and HUVEC (3x10⁵) were incubated for 72 hours at 37°C on supports of semipermeable membrane with pores having 1-µm diameter so as to form a continuous mono-layer wholly impermeable to the substance used as control, the fluorescein isothiocyanate (FITC) dextran. The vasal permeability was evaluated with the kit "*The Millipore*^{®} *In Vitro Vascular Permeability Assay".*

The monolayer was treated 8 hours with a permeability inducer, the *Vascular Endothelial Growth Factor* (VEGF). At the concentration of 50 ng/ml VEGF induced an increase in permeability by 36% in HMVEC line and by 42% on HUVEC cells: increases comparable to those highlightable in the CVI clinical picture. Afterwards, the two monolayers were incubated for 8 hours with concentrations of 100 ng/ml of: (i) Sulodexide + Diosmin (Sul+Dios), (ii) Sulodexide + Hesperidin (Sul+Esp), (iii) Sulodexide + Troxerutin (Sul + Trox), (iv) Diosmin, (v) Hesperidin, (vi) Troxerutin and (vii) Sulodexide, administered singularly.

The tested concentration was selected based upon preliminary experiments aimed at excluding possible interferences on vitality and cellular proliferative capability. FITC-dextran was then introduced. Through fluorometer the endothelial permeability evaluated as concentration of FITC-dextran, which had exceeded the barrier formed by the cellular monolayer, was tested.

### Results

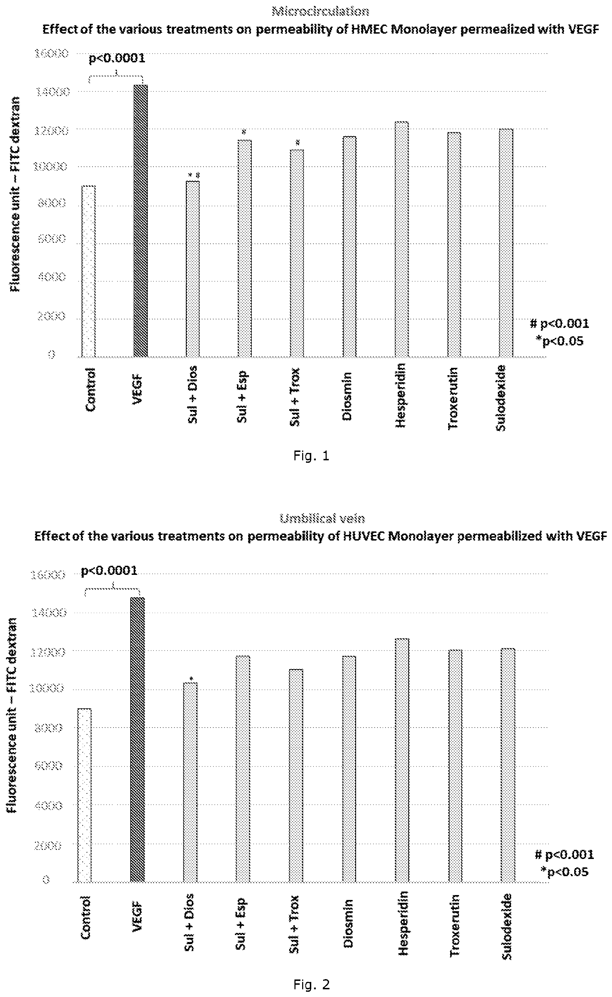

At the end of the study, it was highlighted that:
- the treatment for 8 hours with VEGF induced a statistically significant increase in the permeability with respect to the not treated control (Ground) for both tested cellular models (p<0.0001).
- Surprisingly, the association Sulodexide + flavonoids (that is, the compositions from (i) to (iii)), even if comprising lower concentrations of active principles than those of the active principles administered singularly, restored the increase in the vasal permeability VEGF- induced in both endothelial models (p<0.0001) with a statistically significant difference with respect to the flavonoids and sulodexide administered singularly (p<0.01).
- Among the various tested compositions comprising Sulodexide + flavonoids, the composition comprising Sulodexide + Diosmin (Sul+Dios) highlighted a surprisingly greater effect on the reduction in the vasal hyperpermeability if compared to the one exerted by the other compositions (statistically significant difference p<0.05; the results are illustrated in Figure 1 and in Figure 2).
- The comparison with the administrations of the singularly tested various molecules brought to the light the synergic effect of the association Sulodexide + Diosmin on the reduction in the altered vasal hyperpermeability. Without being bound to the theory, such result can be connected to the synergy between the action mechanism of Sulodexide such as the restoration of the membrane glycocalyx, the reduction in the vasal hyperpermeability and the reduction in the infiltration of the inflammatory molecules, and the action mechanism of Diosmin which, through the inhibition of catechol-o-methyl transferase (COMT) enzyme, increases the available adrenaline levels and then its vasoconstriction effect directed against oedema.

### Example 2. In vivo study on animal model of chronic venous insufficiency

The treatments described in the example 1 were tested in an experimental study performed on an animal model of chronic venous insufficiency, which allowed to evaluate *in vivo,* through oral administration, the effect of such compositions on the microvascular alterations depending upon the increase in the venous pressure. 18 male hamsters (*Mesocricetus auratus*) weighing 85 - 120 gr were subjected to venous ligation and randomized in 9 groups, respectively treated by oral route with composition based on:
1. 0.25% Sulodexide + 4.5% Diosmin (Sul+Dios) in 10% lactose solution;
2. 0.25% Sulodexide + 4.5% Hesperidin (Sul+Esp) in 10% lactose solution;
3. 0.25% Sulodexide + 4.5% Troxerutin (Sul+Trox) in 10 % lactose solution;
4. 8% Diosmin in 10% lactose solution;
5. 8% Hesperidin in 10% lactose solution;
6. 8% Troxerutin in 10% lactose solution;
7. 0.5% Sulodexide in 10% lactose solution;
8. 10% lactose solution (Placebo Group);
9. Healthy check, not subjected to venous ligation and not treated.

After anaesthesia by intraperitoneal injection (xylazine 10 mg/kg + ketamine 200 mg/kg), the animals were subjected to inguinal incision and subsequently to venous ligation, by using suture threads, at the level of the third of the right external iliac vein. Such experimental model of chronic venous insufficiency generates in the animal the increase in the inflammatory process in the rear limbs correlated to a significant increase in the leukocyte-endothelium interaction, micro-angiopathy and decrease in the tissue blood perfusion, decrease in the functional capillary density (FCD), increase in the venular diameter and compromised functionality of the vascular smooth muscle cells.

All formulations to be tested were dissolved in 10% lactose solution and administered with oral probe with volume of 0.2 mL/100 gr of body weight and at the concentration of 100 mg/kg/day for two daily administrations. The various treatments were administered 2 weeks before and for 6 weeks after the venous ligation.

With intravital microscopy connected to a camera, microvascular alterations were evaluated such as: arteriolar and venular diameter, functional capillary density (with fluorescein isothiocyanate-dextran - FITC-dextran), the marked circulating leukocytes and the rolling as well as the adhesion of the leukocytes to endothelium through video-recordings (a leukocyte was considered rolling when it was in contact with the venular wall and it had a lower speed than the circulating erythrocytes and considering adherent when it was immobilized in a same position for at least 30 seconds).

### Results

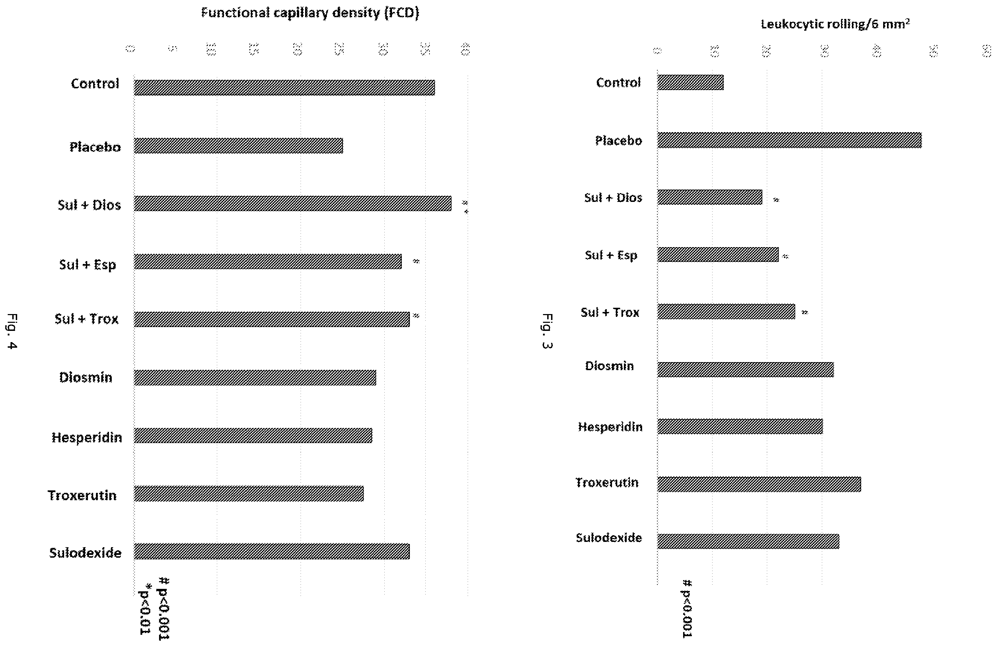

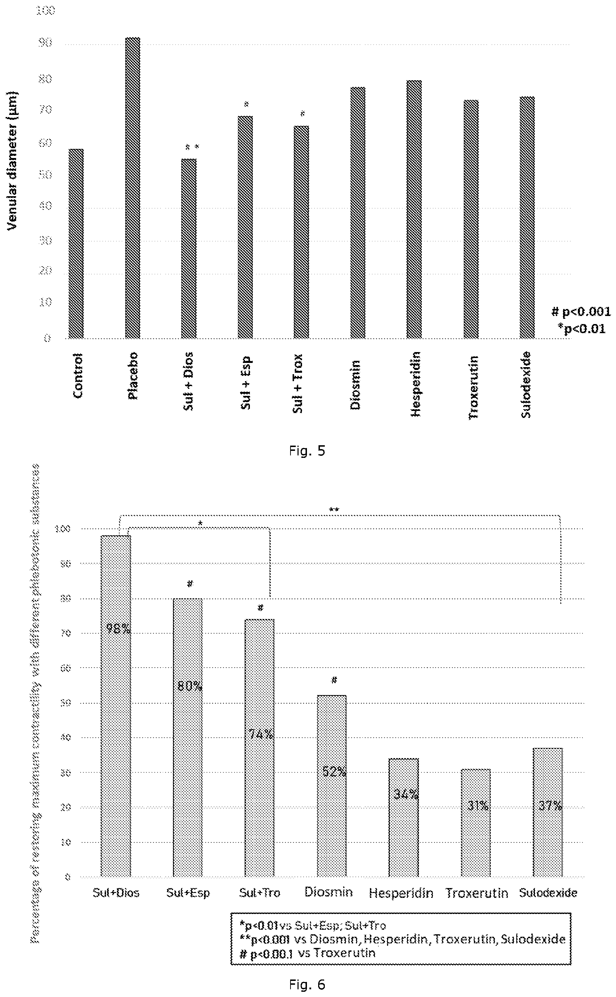

The results confirmed that:
- all animals subjected to venous ligation had an inflammatory status, expressed as number of circulating leukocytes significantly higher than the control group, a decrease in the functional capillary density (FCD) and an increase in the venular diameter (p<0.001).
- All tested treatments involved a decrease in the circulating leukocytes; surprisingly, the decrease obtained in the groups treated with the composition comprising Sulodexide+ flavonoids resulted to be higher than the one obtained with Diosmin, Hesperidin, Troxerutin and Sulodexide administered singularly (p< 0.001), even if lower concentrations of active principles than the concentrations of the singularly tested active principles were used. A higher, even if not statistically significant, decrease was highlighted in the group treated with Sul+Dios with respect to the one highlighted with the other two associations (Figure 3).
- One surprisingly found a less decrease in FCD in the groups treated with the composition comprising Sulodexide + flavonoids with a statistically significant difference with respect to the groups treated with Diosmin, Hesperidin, Troxerutin and Sulodexide administered singularly (p< 0.001). The group treated with Sul+Dios demonstrated to reduce and prevent significantly FCD decrease with a statistically significant difference with respect to the other associations Sulodexide + flavonoids (p<0.01) (Figure 4).
- The venular diameter resulted to be significantly reduced in the group treated with the composition comprising Sulodexide + flavonoids with respect to the other treatments (p<0.001). The group treated with Sul+Dios reduced in a statistically significant way the venular diameter with respect to all other treatment groups (p<0.01) (Figure 5).

The oral treatment with a composition comprising Sulodexide + flavonoids demonstrated to be effective in restoring the microvascular alterations in this animal model of chronic venous insufficiency, with a higher effectiveness with respect to the same evaluated molecules, administered singularly. A more marked effectiveness was surprisingly found in the group treated with Sulodexide + Diosmin. Such evidence highlights the action synergism of the treatment Sulodexide+Diosmin, by confirming what already seen with the preceding *in vitro* model.

### Example 3. Ex vivo study on human saphenous veins

By virtue of the obtained experimental results a study on saphenous veins was implemented which surveyed the direct effect on the venous tone of the composition comprising Sulodexide + flavonoids, by means of evaluation of the effect on the contractile response to the noradrenalin of the compositions comprising Sulodexide + Diosmin (Sul+Dios), Sulodexide + Hesperidin (Sul+Esp) and Sulodexide + Troxerutin (Sul+Trox) (all in concentration respectively equal to 0.25% of sulodexide and 4.5% of flavonoide, expressed by weight with respect to the total weight of the composition). Such effect was compared with the effect of the active principles 8% Diosmin, Hesperidin, Troxerutin and 0.5% Sulodexide, administered singularly.

The vein fragments were collected from subjects affected by varicose disease, subjected to local anaesthesia, through simple incision of the varicose area of the saphenous vein or collateral ones. The fragments were kept for 24-48 hours in solution of Krebs-Henseleit at 4°C and pH 7. The 3-4-mm long venous rings were subjected to isometric contraction (tension of about 1 gr); after a stabilization step the ring reactivity to the hyper-potassic stimulation [50 mM for about 2 hours] was validated. Under conditions of constant hyperthermia (41°C) the several preparations under analysis were incubated for 30 minutes at the concentration of 10⁻⁴M; then cumulative concentrations of noradrenalin (10⁻⁶ M) were introduced. For each ring, the results were expressed in maximum response percentage to noradrenalin (agonist) and the dose/effect curves were detected at the temperature of 41°C.

The statistical analysis of the results demonstrated that the association Sulodexide + flavonoids restores the venous tone at 41°C by strengthening the contractile response to noradrenalin of the venous rings with a difference, in the restoration percentage, statistically significant with respect to the various substances administered singularly (p<0.001), even if comprising significantly lower concentrations of active principles than the concentrations of the same active principles tested singularly. Moreover, the association Sul + Dios demonstrated a greater action on the venous tone with a, surprisingly, statistically significant difference in percentage of venous tone restoration with respect to the other associations (p<0.01). Figure 6 shows the detail of the obtained results.

The results confirm that associating Sulodexide to the flavonoids (diosmin, hesperidin and troxerutin) guarantees a synergic action on the venous tone. Among the various tested compositions, the combination Sulodexide + Diosmin demonstrated the most marked effect: without wanting to be linked to theory, one assumes that such result derives from the synergic effect of the two active principles Sulodexide and Diosmin which by means of two different action mechanisms act, both, on the venous tone. Sulodexide exerts its venous active action by increasing the vasal contractility thanks to the inhibition of the metalloproteinases 2 and 9, and Diosmin its phlebotonic action through inhibition of Catechol-o methyl transferase enzyme and the consequent decrease in the metabolism of noradrenalin (NA); the increase in the NA concentration available at level of the vasal adrenergic receptors thus extends the vasoconstrictive and phlebotonic effect.

## Claims

1. An oral composition comprising sulodexide and one or more flavonoids.

2. The oral composition according to claim 1, wherein said one or more flavonoids are flavones or derivatives thereof.

3. The oral composition according to claims 1 or 2, wherein said one or more flavones or derivatives thereof are selected from diosmin, hesperidin, troxerutin, oxerutin, quercetin and hesperitin.

4. The oral composition according to any one of claims 1 to 3, comprising sulodexide and diosmin.

5. The oral composition according to any one of claims 1 to 4, in the form of tablet, hard capsule, soft capsule, suspension, emulsion or solution.

6. The oral composition according to any one of claims 1 to 5, wherein said sulodexide is in an amount comprised between 21 and 29 mg, preferably equal to 25 mg.

7. The oral composition according to any one of claims 1 to 6, wherein said one or more flavonoids are in an amount comprised between 380 and 520 mg, preferably equal to 450 mg.

8. The oral composition according to any one of claims 1 to 7, comprising a plant extract comprising said one or more flavonoids.

9. The oral composition according to any one of claims 1 to 8, wherein said sulodexide and said one or more flavonoids are the active principles of the composition.

10. The oral composition according to any one of claims 1 to 9, further comprising one or more pharmaceutically acceptable excipients.

11. The oral composition according to any one of claims 1 to 10, for use in the treatment of vascular diseases and/or in the prevention of complications associated with or caused by these diseases.

12. The oral composition for use according to claim 11, wherein said vascular diseases comprise chronic venous insufficiency and/or symptoms or disorders associated with or caused by chronic venous insufficiency.

## Patentansprüche

1. Orale Zusammensetzung, umfassend Sulodexid und ein oder mehrere Flavonoide.

2. Orale Zusammensetzung nach Anspruch 1, wobei das eine oder die mehreren Flavonoide Flavone oder Derivate davon sind.

3. Orale Zusammensetzung nach Anspruch 1 oder 2, wobei das eine oder die mehreren Flavone oder Derivate davon ausgewählt sind aus Diosmin, Hesperidin, Troxerutin, Oxerutin, Quercetin und Hesperitin.

4. Orale Zusammensetzung nach einem der Ansprüche 1 bis 3, umfassend Sulodexid und Diosmin.

5. Orale Zusammensetzung nach einem der Ansprüche 1 bis 4 in der Form einer Tablette, einer Hartkapsel, einer Weichkapsel, einer Suspension, einer Emulsion oder einer Lösung.

6. Orale Zusammensetzung nach einem der Ansprüche 1 bis 5, wobei das Sulodexid in einer Menge, die zwischen 21 und 29 mg umfasst ist, vorzugsweise gleich 25 mg, vorliegt.

7. Orale Zusammensetzung nach einem der Ansprüche 1 bis 6, wobei das eine oder die mehreren Flavonoide in einer Menge, die zwischen 380 und 520 mg umfasst ist, vorzugsweise gleich 450 mg, vorliegen.

8. Orale Zusammensetzung nach einem der Ansprüche 1 bis 7, umfassend einen Pflanzenextrakt, umfassend das eine oder die mehreren Flavonoide.

9. Orale Zusammensetzung nach einem der Ansprüche 1 bis 8, wobei das Sulodexid und das eine oder die mehreren Flavonoide die Wirkstoffe der Zusammensetzung sind.

10. Orale Zusammensetzung nach einem der Ansprüche 1 bis 9, ferner umfassend einen oder mehrere pharmazeutisch verträgliche Hilfsstoffe.

11. Orale Zusammensetzung nach einem der Ansprüche 1 bis 10 zur Verwendung in der Behandlung von Gefäßerkrankungen und/oder in der Vorbeugung von Komplikationen, die mit diesen Erkrankungen verknüpft sind oder durch diese verursacht werden.

12. Orale Zusammensetzung zur Verwendung nach Anspruch 11, wobei die Gefäßerkrankungen chronische venöse Insuffizienz und/oder Symptome oder Störungen, die mit chronischer venöser Insuffizienz verknüpft sind oder durch diese verursacht werden, umfassen.

## Revendications

1. Composition orale comprenant du sulodexide et un ou plusieurs flavonoïdes.

2. Composition orale selon la revendication 1, dans laquelle ledit ou lesdits flavonoïdes sont des flavones ou des dérivés de celles-ci.

3. Composition orale selon les revendications 1 ou 2, dans laquelle ladite ou lesdites flavones ou dérivés de celles-ci sont choisis parmi la diosmine, l'hespéridine, la troxérutine, l'oxérutine, la quercétine et l'hespéritine.

4. Composition selon l'une quelconque des revendications 1 à 3, comprenant du dulodexide et de la diosmine.

5. Composition orale selon l'une quelconque des revendications 1 à 4, sous forme de comprimé, de gélule, de capsule molle, de suspension, d'émulsion ou de solution.

6. Composition orale selon l'une quelconque des revendications 1 à 5, dans laquelle ledit sulodexide est présent en une quantité comprise entre 21 et 29 mg, de préférence égale à 25 mg.

7. Composition orale selon l'une quelconque des revendications 1 à 6, dans laquelle ledit ou lesdits flavonoïdes sont présents en une quantité comprise entre 380 et 520 mg, de préférence égale à 450 mg.

8. Composition orale selon l'une quelconque des revendications 1 à 7, comprenant un extrait végétal comprenant ledit ou lesdits flavonoïdes.

9. Composition orale selon l'une quelconque des revendications 1 à 8, dans laquelle ledit sulodexide et ledit ou lesdits flavonoïdes sont les principes actifs de la composition.

10. Composition orale selon l'une quelconque des revendications 1 à 9, comprenant en outre un ou plusieurs excipients pharmaceutiquement acceptables.

11. Composition orale selon l'une quelconque des revendications 1 à 10, pour une utilisation dans le traitement de maladies vasculaires et/ou dans la prévention de complications associées à ou causées par ces maladies.

12. Composition orale pour une utilisation selon la revendication 11, dans laquelle lesdites maladies vasculaires comprennent l'insuffisance veineuse chronique et/ou des symptômes ou troubles associés à ou causés par l'insuffisance veineuse chronique.
